**Europäisches Patentamt**

**European Patent Office**

**Office européen des brevets**

(19)

(11) Veröffentlichungsnummer: **0 150 820**
**B1**

(12)

# EUROPÄISCHE PATENTSCHRIFT

(45) Veröffentlichungstag der Patentschrift:
**12.07.89**

(21) Anmeldenummer: **85100739.3**

(22) Anmeldetag: **25.01.85**

(51) Int. Cl.⁴: **C 07 C 85/12,** C 07 C 87/14,
B 01 J 23/74, B 01 J 23/84,
B 01 J 23/86

(54) **Verfahren zur Herstellung von gesättigten primären Aminen.**

(30) Priorität: **01.02.84 DE 3403376**

(43) Veröffentlichungstag der Anmeldung:
**07.08.85 Patentblatt 85/32**

(45) Bekanntmachung des Hinweises auf die Patenterteilung:
**12.07.89 Patentblatt 89/28**

(84) Benannte Vertragsstaaten:
**BE CH DE FR GB IT LI NL**

(56) Entgegenhaltungen:
**DE-A-2 301 139**
**DE-A-2 654 028**
**FR-A-1 407 414**

(73) Patentinhaber: **BASF Aktiengesellschaft, Carl-Bosch- Strasse 38, D-6700 Ludwigshafen (DE)**

(72) Erfinder: **Frank, Gerhard, Dr., Heidelberger Strasse 11, D-6945 Hirschberg (DE)**
Erfinder: **Neubauer, Gerald, Dr., Mozartstrasse 24, D-6940 Weinheim (DE)**
Erfinder: **Duffner, Paul, Dr., Bozener Strasse 14, D-6700 Ludwigshafen (DE)**
Erfinder: **Wilfinger, Hans Joerg, Dr., Buschstrasse 11, D-6707 Schifferstadt (DE)**

EP 0 150 820 B1

2

### Beschreibung

Bei der Herstellung von Aminen durch Hydrieren von Nitrilen, z.B. Hexamethylendiamin aus Adiponitril, werden Kobalt enthaltende Katalysatoren wegen ihrer hohen Selektivität besonders bevorzugt verwendet. Solche Verfahren sind beispielsweise aus den DE-PSen 1 072 972 und 1 259 899 bekannt. Die Lebensdauer der verwendeten Kobaltkatalysatoren entspricht jedoch nicht mehr den technischen Anforderungen. Die verwendeten Katalysatoren haben den Nachteil, daß sie im Laufe ihres Gebrauchs zerfallen, wodurch ihre Wirkungsweise beeinträchtigt wird. Aus der FR-PS-1 407 414 ist auch schon bekannt, daß man Kobaltkatalysatoren, die durch Verpressen von Kobaltoxid mit Graphit erhalten worden sind, für die Hydrierung von Adipodinitril zu Hexamethylendiamin verwendet. Solche Katalysatoren verlieren beim Gebrauch rasch ihre Härte und zerfallen, wodurch ihre Dauergebrauchsfähigkeit sehr beschränkt ist. Aus der DE-OS-2 654 028 sind auch bereits geformte Katalysatormassen, die metallisches Kobalt, Alkali- und Erdalkalioxide sowie Graphit enthalten, bekannt. Diese Katalysatoren führen bei der Hydrierung zu Adipodinitril jedoch zur vermehrten Bildung von cyclischen Produkten.

Es war die technische Aufgabe gestellt, geformte Kobalt und/oder Nickel enthaltende Katalysatormassen zur Verfügung zu stellen, die sich durch Hohe Aktivität und lange Lebensdauer, insbesondere hohe Druckhärte, mechanische Beständigkeit und Abriebfestigkeit auszeichnen.

Diese Aufgabe wird gelöst in einem Verfahren zur Herstellung von gesättigten primären Aminen durch Umsetzung von Nitrilen mit Wasserstoff bei erhöhter Temperatur und unter erhöhtem Druck in Gegenwart von Ammoniak und Kobalt und/oder Nickel sowie Gleitmittel enthaltenden Katalysatoren, wobei man geformte Katalysatormassen mit einer Druckhärte von $\geq 300$ kp/cm², enthaltend metallische Kobalt- und/oder Nickelteilchen, die aus Kobalt- und/oder Nickeloxidteilchen mit einem Gehalt von weniger als 0,1 Gew.-% an Alkali- und Erdalkalioxiden durch Reduktion mit Wasserstoff bei einer Temperatur $\leq 500°C$ erhalten worden sind, und ein Gleitmittel, verwendet.

Das neue Verfahren hat den Vorteil, daß die verwendeten Katalysatoren eine lange Lebensdauer haben und sich auch nach längerem Gebrauch noch durch überlegene Eigenschaften auszeichnen. Insbesondere hat das neue Verfahren den Vorteil, daß die verwendeten Katalysatoren auch nach langem Gebrauch hohe Druckhärte und mechanische Beständigkeit sowie Abriebfestigkeit aufweisen. Das neue Verfahren hat ferner den Vorteil, daß es mit hoher Selektivität verläuft und wenig Nebenprodukte anfallen, die schwer von den Wertprodukten abtrennbar sind.

Als Ausgangsstoffe verwendet man bevorzugt aliphatische, cycloaliphatische oder aromatische Nitrile mit bis zu 20 Kohlenstoffatomen. Die Nitrilgruppe kann ein- oder mehrmals, z. B. 1- bis 4-mal, im Molekül vorkommen. Für die Hydrierung eignen sich gesättigte oder olefinisch ungesättigte Nitrile. Sie können auch unter Reaktionsbedingungen inerte Substituenten haben, wie über Etherbrücken gebundene Alkylreste mit 1 bis 4 Kohlenstoffatomen oder aromatisch gebunde Halogenatome. Besonders bevorzugte Ausgangsstoffe sind Alkannitrile oder Alkandinitrile mit 3 bis 18 Kohlenstoffatomen. Geeignete Nitrile sind beispielsweise Propionitril, Acetonitril, Acrylnitril, Benzylcyanid, Benzonitril, Glutarsäuredinitril, Methylglutarsäuredinitril, Adipinsäuredinitril, But-2-en-dinitril-1,4.

Besondere technische Bedeutung hat das Verfahren bei der Hydrierung von Adipinsäuredinitril zu Hexamethylendiamin erlangt.

Die Umsetzung führt man im allgemeinen unter Drücken von 100 bis 400 bar, vorzugsweise 200 bis 300 bar, durch. Vorteilhaft hält man Temperaturen von 80 bis 140°C, vorzugsweise von 100 bis 120°C, ein.

Die Umsetzung wird in Gegenwart von Ammoniak durchgeführt. Es hat sich bewährt, wenn das Volumenverhältnis von Nitril zu Ammoniak von 1 : 2 bis 1 : 20, vorzugsweise 1 : 6 bis 1 : 12 beträgt. Es ist auch möglich, Ammoniak teilweise durch zurückgeführtes rohes Hydriergemisch, das im wesentlichen aus dem herzustellenden Amin und Ammoniak besteht, zu ersetzen.

Erfindungsgemäß verwendet man geformte Katalysatormassen mit einer Druckhärte von $\geq 300$ kp/cm², enthaltend metallische Kobalt- und Nickelteilchen, die aus Kobalt- und/oder Nickeloxidteilchen durch Reduktion mit Wasserstoff bei einer Temperatur $\leq 500°C$ erhalten worden sind, und ein Gleitmittel. Vorteilhaft weisen die metallischen Kobalt- und/oder Nickelteilchen einen Reduktionsgrad von $\geq 80\%$, insbesondere $\leq 95\%$ auf. Unter Reduktionsgrad ist der Anteil des verfügbaren Kobalts und/oder Nickels in % zu verstehen, der in metallischer Form vorliegt.

Der Gehalt an Alkali- und/oder Erdalkalioxiden beträgt weniger als 0,1 Gew.-%.

Die verwendeten geformten Katalysatormassen enthalten ferner Gleitmittel, z. B. anorganische Stoffe mit Gitterstruktur wie Talkum oder Graphit. Vorteilhaft enthalten die Katalysatoren 1 bis 5 Gew.-% an Gleitmittel, bezogen auf die gesamte Katalysatormasse aus aktiver Masse enthaltend metallische Kobalt- und/oder Nickelteilchen sowie Gleitmittel. Besonders bewährt hat sich Graphit als Gleitmittel. Die erfindungsgemäßen Katalysatormassen haben eine Druckhärte von $\leq 300$ kp/cm², insbesondere von 350 bis 1500 kp/cm².

Bevorzugt werden Kobalt- und/oder Nickeloxidteilchen mit einer Teilchengröße von 0,1 bis 5 μm, insbesondere von 0,2 bis 2 μm verwendet. Die als Ausgangsstoffe verwendeten Kobalt- und/oder Nickeloxidteilchen können weitere aktivierende Zusätze, wie Mangan-, Chrom- und /oder Kupferoxid, ferner Pyro- und/oder Polyphosphorsäure enthalten. Bevorzugt enthalten die Oxidteilchen 80 bis 99 Gew.-% Nickel und/oder

2

Kobalt in Form ihrer Oxide, berechnet als Metall und 0,5 bis 10 Gew.-% Mangan, Chrom und/oder Kupfer in Form ihrer Oxide, berechnet als Metall. Sofern der Katalysator mit Pyro- oder Polyphosphorsäure modifiziert sein soll, enthalten die oxidischen Ansgangsteilchen 0,5 bis 10 Gew.-% Poly- und/oder Pyrophosphorsäure. Es versteht sich, daß die daraus resultierende katalytisch aktive Masse, abzüglich Gleitmittel, die entsprechende Zusammensetzung im fertigen Katalysator hat. Es ist davon auszugehen, daß im fertigen Katalysator Kupfer als Metall vorliegt, während nicht bekannt ist, in welcher Form Mangan und/oder Chrom im fertigen Katalysator vorliegen.

Die fertigen Katalysatoren bestehen somit im wesentlichen aus metallischen Kobalt- und/oder Nickelteilchen, geringen Mengen Kobalt- und/oder Nickeloxid entsprechend dem Reduktionsgrad, gegebenenfalls den vorgenannten Zusätzen und einem Gleitmittel.

Die erfindungsgemäßen Katalysatoren sind geformt z. B. als Kugeln, Tabletten oder Stränge.

Bei der Herstellung der Oxidteilchen geht man in der Regel von wäßrigen Lösungen von Kobalt- und/oder Nickelsalzen aus. Falls aktivierende Zusätze wie Mangan, Chrom und/oder Kupfer mitverwendet werden sollen, benutzt man zusätzlich wäßrige Lösungen von Salzen dieser Metalle. Geeignete Salze sind beispielsweise Nitrate, Sulfate, Chloride oder Salze mit niedrigen Fettsäuren wie Essigsäure. Soll der Katalysator Pyro- oder Polyphosphorsäure enthalten, so wird Phosphorsäure mitverwendet, die bei der weiteren Verarbeitung in Pyro- oder Polyphosphorsäure übergeht. Die Ausgangslösungen werden zweckmäßig zunächst im sauren Bereich, etwa im pH-Bereich von 1 bis 2, vereinigt. Dann fällt man in der Regel mittels wäßriger Alkalilauge, z.B mit 10 bis 25 Gew.-%, oder wäßriger Alkalicarbonatlauge, z. B. mit 5 bis 25 Gew.-%, ein Gemisch der Metalloxidhydroxide und Carbonate ggf. zusammen mit den Säurebestandteilen aus. Es ist auch möglich, die wäßrigen Lösungen der Metallverbindungen in die alkalische Fällösung einlaufen zu lassen. Es hat sich als vorteilhaft erwiesen, die Mengenverhältnisse von saurer Lösung und alkalischem Fällmittel so zu wählen, daß sich im Reaktionsgemisch am Ende ein pH-Wert von 7,0 bis 7,5 einstellt. Das ausgefallene Oxid-, Hydroxid- und/oder Carbonatgemisch wird abfiltriert, mit Wasser von Fremdsalzen freigewaschen und getrocknet. Anschließend wird das Gemisch durch Erhitzen in die entsprechenden Oxide übergeführt. Im allgemeinen sind hierfür Temperaturen von 250 bis 500°C ausreichend. Die so erhaltene Masse wird dann z. B. auf die geeignete Teilchengröße wie vorgenannt gemahlen. Nach einer vorteilhaften Arbeitsweise wird die oxidische Masse nach Anteigen, z. B. mit Wasser, zu Strängen verformt und die so erhaltenen Formkörper noch einmal bei einer Temperatur von 300 bis 800°C getempert und anschließend auf die vorgenannte Teilchengröße gemahlen. Der Gehalt an Alkali- und/oder Erdalkalioxiden beträgt weniger als 0,1 Gew.%.

Die Kobalt- und/oder Nickeloxid enthaltenden Teilchen werden mittels Wasserstoff z.B. in der Wirbelschicht im Drehrohrofen oder vorzugsweise in einem gerührten Festbett, z. B. bei einer Temperatur von 250 bis 500°C, insbesondere 300 bis 450°C, z. B. innerhalb von 3 bis 36 Stunden reduziert. Vorteilhaft verwendet man einen trockenen Wasserstoffstrom, der frei von wesentlichen Mengen Wasser ist, wobei man eine relativ große Wasserstoffströmungsgeschwindigkeit einhält. Es hat sich bewährt, wenn man mindestens einen 60fachen Wasserstoffüberschuß anwendet. Vorteilhaft reduziert man solange bis ein Reduktionsgrad von ≧ 80 %, insbesondere ≧ 95 %, erreicht ist.

Anschließend werden die Metallteilchen durch Passivierung stabilisiert. Hierunter versteht man das Umhüllen der Metallteilchen mit einer Oxidschicht durch kontrollierte Oxidation, um die durch die große freie Oberfläche der kleinen Teilchen bedingte Pyrophorität zu beseitigen. Dies wird durch Behandeln mit einem molekularen Sauerstoff enthaltenden Inertgas erreicht. Vorteilhaft verwendet man hierzu 0,1 bis 1,0 Vol.-% molekularen Sauerstoff enthaltende Inertgase. Inertgase sind beispielsweise Stickstoff oder Edelgase. Besonders bewährt hat es sich, wenn man ein Stickstoff/Luftgemisch verwendet. Die Passivierung wird z. B. erreicht durch Überleiten eines Stickstoff/Luftgemisches unter genauer Einhaltung einer vorzugsweise 100°C, insbesondere 80°C, nicht übersteigenden Temperatur, z. B. von 65 bis 75°C über das Metallpulver. Nach der Passivierung soll der Reduktionsgrad nicht unter 80 %, vorzugsweise nicht unter 90 % liegen. Die stabilisierten Kobalt- und/oder Nickelteilchen haben eine Größe von 0,05 bis 2 μm insbesondere von 0,2 bis 1,2 μm.

Die so passivierten metallischen Kobalt- und/oder Nickelteilchen werden mit einem inerten Gleitmittel, z. B. an organischen Stoffen, mit Gitterstruktur wie Talkum oder Graphit, insbesondere Graphit, vermischt. Hierbei wendet man vorteilhaft 1 bis 5 Gew.-% Gleitmittel, bezogen auf Metallteilchen enthaltender Masse und Gleitmittel an. Das Gemisch aus passivierten Kobalt- und/oder Nickelteilchen und Gleitmitteln wird vorteilhaft unter Stickstoffabdeckung zu Formkörpern verarbeitet, z. B. zu Tabletten verpreßt. Die Druckhärte der Formkörper ist zweckmäßig größer ≧ 300 kp/cm². 

Die so erhaltenen Formkörper werden durch Behandeln mit Wasserstoff in relativ großem Überschuß, z. B. dem 60fachen Überschuß in Abwesenheit von wesentlichen Mengen Wasser bei einer Temperatur ≦ 500°C, z. B. von 250 bis 500°C insbesondere 300 bis 360°C bei Atmosphärendruck oder unter erhöhtem Druck, z. B. bis zu 150 bar aktiviert. Hierbei soll ein Reduktionsgrad von vorteilhaft ≧ 95 % erreicht werden. Durch die Aktivierung steigt die Druckhärte der Formkörper z. B. von 300 auf 600 bis 800 kp/cm².

Das Prinzip der verwendeten geformten Katalysatormassen besteht somit darin, daß man Kobalt- und/oder Nickeloxid enthaltende Teilchen zunächst zu Metallteilchen reduziert, diese mit molekularen Sauerstoff enthaltenden Inertgasen passiviert, um die Pyrophoritität aufzuheben, die passivierten metallisches Kobalt oder Nickel enthaltende Teilchen mit Gleitmitteln zu Formkörpern verpreßt und die so erhaltenen Formkörper durch Reduktion mit Wasserstoff aktiviert.

Es hat sich besonders bewährt, wenn man die fertigen aktivierten geformten Katalysatormassen mindestens noch einmal, durch Behandeln mit molekularen Sauerstoff enthaltendem Inertgas, wie vorgehend beschrieben, passiviert, wobei ein Reduktionsgrad von 80 % nicht unterschritten werden soll und anschließend wiederum durch Behandeln mit Wasserstoff bei einer Temperatur $\leq$ 500°C, vorzugsweise 300 bis 360°C, wie vorgehend beschrieben aktiviert. Dieser Vorgang wird zweckmäßig 1 bis 5-mal, insbesondere 1- bis 4-mal, wiederholt. Hierbei steigt die Druckhärte mit jedem Zyklus und erreicht Werte von 800 bis 1300 kp/cm$^2$.

Die erfindungsgemäß verwendeten geformten Katalysatormassen zeichnen sich durch eine hohe mechanische Stabilität aus, die dadurch erreicht wird, daß man die Verformung der Formkörper zusammen mit Gleitmitteln nicht auf der Stufe der Oxide vornimmt, sondern erst nach deren Reduktion zu Kobalt- und/oder Nickelteilchen und nachfolgender Passivierung. Geht man von Formlingen aus, die aus Kobalt- und/oder Nickeloxidteilchen und Gleitmitteln hergestellt und anschließend aktiviert worden sind, so geht die Druckhärte der Formlinge z. B. von 300 kp/cm$^2$ auf $\leq$ 25 kp/cm$^2$ nach Erreichen eines Reduktionsgrades von 95 % zurück. Die Lebensdauer solcher Katalysatoren wird hierdurch erheblich erniedrigt.

Die Hydrierung der Nitrile kann absatzweise erfolgen, vorteilhaft führt man die Umsetzung kontinuierlich durch, z. B. in Rieselfahrweise an fest angeordneten geformten Katalysatormassen.

Die nach dem Verfahren der Erfindung erhältlichen Amine eignen sich zur Herstellung von Stabilisatoren. Hexamethylendiamin, das nach dem Verfahren der Erfindung erhalten wird, ist ein wichtiges Ausgangsprodukt zur Herstellung von Polyamid-6,6.

Das Verfahren nach der Erfindung sei an folgenden Beispielen veranschaulicht.

**Beispiel 1**

4480 g Kobaltnitrat Co(NO$_3$)$_2$ x 6 H$_2$O, 261 g Manganannitrat Mn(NO$_3$)$_2$ x 6 H$_2$O und 47 g Phosphorsäure 85 gew.-%-ig werden in 10 l Wasser gelöst. Diese Lösung läßt man in eine Lösung von 1900 Natriumcarbonat Na$_2$Co$_3$ in 10 l Wasser langsam unter Rühren einlaufen. Nach beendetem Zulauf der Metallsalzlösung ist der pH-Wert des Gemisches auf 7,0 gefallen. Nach dem Erkalten wird der Niederschlag durch Absaugen getrennt und solange mit Wasser gewaschen bis der Niederschlag frei von Natriumionen ist. Der Filterkuchen wird getrocknet und anschließend auf 300°C erhitzt bis die Masse frei von Carbonat ist. Das Oxidgemisch wird dann mit soviel Wasser angeteigt, daß eine knetbare Masse entsteht. Diese wird in einer Presse zu Strängen verformt. Die Stränge werden dann bei 450°C im Muffelofen für 24 Stunden getempert. Die erhaltenen Stränge werden zu Teilchen einer Größe von 0,1 bis 1,2 μm gemahlen.

600 kg der so hergestellten Kobaltoxid enthaltenden Teilchen werden in einem gerührten Festbett bei 400°C 38 Stunden mit 400 Nm$^3$/h Wasserstoff zu metallischem Kobalt mit einem Reduktionsgrad, bezogen auf Kobalt 95 % reduziert (stöchiometrischer Wasserstoffüberschuß 64). Anschließend wird in einem Stickstoff-Luft-Gemisch das pyrophore Metallpigment bei einer Temperatur von 60°C mit einer stabilisierenden Oxidschicht überzogen und passiviert, wobei der Reduktionsgrad nicht unter 90 % absinken soll.

Zur Herstellung von geformten Massen mit einem Durchmesser von 5 mm und einer Hohe von 4 mm wird das passivierte pulverförmige Metallpigment mit 2 Gew.-% Graphit vermischt und unter Stickstoffabdeckung tablettiert. Die Druckhärte der Tabletten beträgt 300 kp/cm$^2$.

In einem Hochdruckgefäß werden 350 ml der so hergestellten Formkörper bei 360°C unter einem Druck von 150 bar mit hohem Wasserüberschuß für 24 Stunden zum Zweck der Aktivierung behandelt. Der Wasserstoff wird dabei im Kreis über einen Kondensator zur Abscheidung des Reduktionswassers geführt. Die so erhaltene geformte Katalysatormasse hat einen Reinheitsgrad von 98 % und eine Druckhärte von 620 kp/cm$^2$. Anschließend wird die geformte Katalysatormasse durch Behandeln mit einem Gemisch aus Stickstoff mit einem Sauerstoffgehalt von 0,5 Vol.-% bei einer Temperatur von 60°C passiviert, wobei der Reduktionsgrad 90 % nicht unterschreitet. Daraufhin wird geformte Katalysatormasse wiederum bei 360°C und unter einem Druck von 150 bar wie oben beschrieben mit Wasserstoff aktiviert. Die so erhaltenen Formlinge haben eine Druckhärte von 950 kp/cm$^2$. Nach der zweiten Wiederholung des Zyklus Passivieren Aktivieren haben die Formkörper eine Druckhärte von 1140 kp/cm$^2$.

Nach dem Abkühlen des Katalysators wird das Reaktionsgefäß in Rieselfahrweise bei 270 bar Wasserstoffdruck stündlich mit einem Gemisch aus 85 l Adipinsäuredinitril, 255 l flüssigem Ammoniak und 350 l Hydriergemisch unter Kreisgasfahrweise des Wasserstoffs (400 Nm$^3$/h) beschickt. Die Temperatur des Zulaufgemischs beträgt 65°C und die des Reaktorausgangs liegt bei 95°C. Es entstehen hot spot-Temperaturen von maximal 100°C. Die gaschromatographische Analyse des rohen Hexamethylendiamins nach Verdampfen des Ammoniaks aus dem Hydriergemisch ergibt 0,02 Gew.-% Hexylamin, 0,05 Gew.-% Azacyclomethan, 0,06 Gew.-% 1,2-Diamino-cyclohexan, 0,003 2-Aminomethylcyclopentylamin und 99,86 Gew.-% Hexamethylendiamin sowie einen Aminocapronitrilgehalt von 0,001 Gew.-%. Der vorwiegend aus Bis-Hexamethylendiamin bestehende Destillationsrückstand liegt bei 0,3 Gew.-%. Es errechnet sich eine Selektivität von 99,5 Hexamethylendiamin. Der Katalysator hatte nach einer Laufzeit von 520 Tagen eine unveränderte Aktivität und Selektivität ohne jegliche Regenerierung. Die geformte Katalysatormasse hatte nach dieser Laufzeit eine Druckhärte von 900 kp/cm².

### Vergleichsbeispiel 1

Das nach Beispiel 1 hergestellte Kobaltoxid wird nach Zusatz von 2 % Graphit zu Tabletten (Durchmesser 5 mm, Höhe 4 mm) verpreßt. Die Druckhärte wird auf 300 kp/cm² eingestellt. Danach werden die Tabletten mit Wasserstoff bei 360°C auf einen Reduktionsgrad von $\geq$ 95 % gebracht. Durch die Reduktion des Kobaltoxids sinkt die Druckhärte der Tabletten drastisch auf Werte $\leqslant$ 25 kp/cm². Die Tabletten zerfallen bei Berührung der zylindrischen Mantelfläche leicht in Scheibchen.

Im Unterschied zu Tabletten, die aus bereits zu Kobalt reduziertem Kobaltoxid hergestellt wurden, kann die mechanische Stabilität dieser Tabletten durch wiederholtes Passivieren und Reduzieren nicht verbessert werden.

### Vergleichsbeispiel 2

Ein Hochdruckgefäß von 2 m Länge und 42 mm Durchmesser wird mit 3 l eines nach Beispiel 1 der DE-OS-2 654 028 hergestellten passivierten Kobaltkatalysator beschickt. Der Katalysator enthält 1,5 Gew.-% Calciumoxid und 2,7 Gew.-% Natriumsalze, berechnet als Natriumoxid. Die Passivierung des Katalysators wird durch Erhitzen in einem Wasserstoffstrom auf 330°C aufgehoben.

Nach dem Abkühlen wird das Hochdruckgefäß in Rieselfahrweise bei 270 bar Wasserstoffdruck stündlich mit 0,4 l Adipodinitril 1,2 l flüssigen Ammoniak sowie 4,5 l rohem Hydriergemisch beschickt. Hierbei hält man eine Temperatur von 140°C ein. Die gaschromatographische Analyse des rohen Hexamethylendiamin nach Verdampfen des Ammoniaks aus dem Hydriergemisch ergibt 99,69 Gew.-% Hexamethylendiamin, 0,06 Gew.-% 1,2-Diaminocyclohexan und 0,12 Gew.-% 2-Aminomethylcyclopentylamin.

## Patentansprüche

1. Verfahren zur Herstellung von gesättigten primären Aminen durch Umsetzen von Nitrilen mit Wasserstoff bei erhöhter Temperatur und unter erhöhtem Druck in Gegenwart von Ammoniak und Kobalt und/oder Nickel, sowie ein Gleitmittel enthaltenden Katalysatoren, dadurch gekennzeichnet, daß man geformte Katalysatormassen mit einer Druckhärte von $\geq$ 300 kp/cm², enthaltend metallische Kobalt- und/oder Nickelteilchen, die aus Kobalt- und/oder Nickeloxidteilchen mit einem Gehalt von weniger als 0,1 Gew.-% Alkali- und Erdalkalioxid durch Reduktion mit Wasserstoff bei einer Temperatur $\leq$ 500°C erhalten worden sind, und ein Gleitmittel, verwendet.

2. Verfahren nach Anspruch 1, dadurch gekennzeichnet, daß die geformten Katalysatormassen eine Druckhärte von 350 bis 1500 kp/cm² haben.

3. Verfahren nach den Ansprüchen 1 und 2, dadurch gekennzeichnet, daß die geformten Katalysatormassen 1 bis 5 Gew.-% Gleitmittel enthalten.

4. Verfahren nach den Ansprüchen 1 bis 3, dadurch gekennzeichnet, daß die geformten Katalysatormassen Graphit als Gleitmittel enthalten.

5. Verfahren nach den Ansprüchen 1 bis 4, dadurch gekennzeichnet, daß die geformten Katalysatormassen Mangan, Chrom und/oder Kupfer als aktivierende Zusätze enthalten.

6. Verfahren nach den Ansprüchen 1 bis 5, dadurch gekennzeichnet, daß die geformten Katalysatormassen einen Gehalt an Pyro- und /oder Polyphosphorsäuren haben.

7. Verfahren nach den Ansprüchen 1 bis 6, dadurch gekennzeichnet, daß man Katalysatoren verwendet, die erhalten worden sind, indem man

a) Kobalt- und/oder Nickeloxidteilchen durch Behandeln mit Wasserstoff zu metallischen Kobalt- und/oder Nickelteilchen reduziert,

b) die metallischen Kobalt- und/oder Nickelteilchen durch Behandeln mit molekularen Sauerstoff enthaltendem Inertgas passiviert,

c) die passivierten Kobalt- und/oder Nickelteilchen mit Gleitmitteln zu Formkörpern preßt und

d) die passivierten Kobalt- und/oder Nickelteilchen sowie Gleitmittel enthaltende Formkörper durch Behandeln mit Wasserstoff bei einer Temperatur von 250 bis 500°C aktiviert.

8. Verfahren nach Anspruch 7, dadurch gekennzeichnet, daß man die Kobalt- und/oder Nickeloxidteilchen bis zu einem Reduktionsgrad von $\geq$ 95 % reduziert.

9. Verfahren nach den Ansprüchen 7 und 8, dadurch gekennzeichnet, daß man beim Passivieren der metallischen Kobalt- und/oder Nickelteilchen einen Reduktionsgrad von 80 % nicht unterschreitet.

10. Verfahren nach den Ansprüchen 7 bis 9, dadurch gekennzeichnet, daß man die Kobalt- und/oder Nickelteilchen sowie Gleitmittel enthaltenden Formkörper zusätzlich mindestens einmal durch Behandeln mit molekularen Sauerstoff enthaltendem Inertgas passiviert und anschließend durch Behandeln mit Wasserstoff bei einer Temperatur von 250 bis 500° C aktiviert.

11. Verfahren nach den Ansprüchen 1 bis 10, dadurch gekennzeichnet, daß man Alkannitrile oder Alkandinitrile mit 3 bis 18 Kohlenstoffatomen als Ausgangsstoffe verwendet.

**Claims**

1. A process for the preparation of a saturated primary amine by reacting a nitrite with hydrogen at elevated temperatures and under superatmospheric pressure in the presence of ammonia and a catalyst containing cobalt and/or nickel and a lubricant, wherein the molded catalyst material used has an indentation hardness of $\geq 300$ kp/cm$^2$ and contains metallic cobalt and/or nickel particles, obtained from cobalt oxide and/or nickel oxide particles containing less than 0.1 % by weight of alkali metal oxide and/or alkaline earth metal oxide by reduction with hydrogen at $\leq 500°$C, and a lubricant.

2. A process as claimed in claim 1, wherein the molded catalyst material has an indentation hardness of from 350 to 1,500 kp/cm$^2$.

3. A process as claimed in either of claims 1 and 2, wherein the molded catalyst material contains from 1 to 5 % by weight of a lubricant.

4. A process as claimed in any of claims 1 to 3, wherein the molded catalyst material contains graphite as the lubricant.

5. A process as claimed in any of claims 1 to 4, wherein the molded catalyst material contains manganese, chromium and/or copper as activating additives.

6. A process as claimed in any of claims 1 to 5, wherein the molded catalyst material contains pyrophosphoric and/or polyphosphoric acid.

7. A process as claimed in any of claims 1 to 6, wherein the catalyst used is obtained by
a) reducing cobalt oxide and/or nickel oxide particles to metallic cobalt and/or nickel particles by treatment with hydrogen,
b) passivating the metallic cobalt and/or nickel particles by treatment with an inert gas containing molecular oxygen,
c) pressing the passivated cobalt and/or nickel particles with a lubricant to give moldings, and
d) activating the moldings containing passivated cobalt and/or nickel particles and lubricant by treatment with hydrogen at from 250 to 500°C.

8. A process as claimed in claim 7, wherein the cobalt oxide and/or nickel oxide particles are reduced until the degree of reduction is $\geq 95$ %.

9. A process as claimed in either of claims 7 and 8, wherein, when the metallic cobalt and/or nickel particles are passivated, the degree of reduction does not fall below 80 %.

10. A process as claimed in any of claims 7 to 9, wherein the moldings containing cobalt and/or nickel particles and lubricant are additionally passivated by treatment with an inert gas containing molecular oxygen and then activated by treatment with hydrogen at from 250 to 500°C, these procedures being carried out one or more times.

11. A process as claimed in any of claims 1 to 10, wherein an alkanenitrile or alkanedinitrile of 3 to 18 carbon atoms is used as a starting material.

**Revendications**

1. Procédé de préparation d'amines primaires saturées par réaction de nitriles avec l'hydrogène à température élevée et sous pression élevée en présence d'ammoniac et de cobalt et/ou de nickel, ainsi que de catalyseurs contenant un lubrifiant, caractérisé en ce qu'on utilise des masses catalytiques façonnées ayant une dureté à la compression $\geq 300$ kp/cm$^2$, contenant des particules de cobalt et/ou de nickel métallique qui ont été obtenues à partir de particules d'oxyde de cobalt et/ou de nickel ayant une teneur en oxydes alcalins et alcalino-terreux de moins de 0,1 % en poids, par réduction par l'hydrogène à une température $\leq 500°$C, et un lubrifiant.

2. Procédé selon la revendication 1, caractérisé en ce que les masses catalytiques façonnées ont une dureté à la compression de 350 à 1500 kp/cm$^2$.

3. Procédé selon les revendications 1 et 2, caractérisé en ce que les masses catalytiques façonnées contiennent de 1 à 5 % en poids de lubrifiant.

4. Procédé selon les revendications 1 à 3, caractérisé en ce que les masses catalytiques façonnées contiennent du graphite comme lubrifiant.

5. Procédé selon les revendications 1 à 4, caractérisé en ce que les masses catalytiques façonnées contiennent du manganèse, du chrome et/ou du cuivre comme additifs d'activation.

6. Procédé selon les revendications 1 à 5, caractérisé en ce que les masses catalytiques façonnées ont une teneur en acide pyro et/ou polyphosphorique.

7. Procédé selon les revendications 1 à 6, caractérisé en ce qu'on utilise des catalyseurs qui ont été préparés comme suit:
a) on réduit des particules d'oxyde de cobalt et/ou de nickel par traitement par l'hydrogène en particules de cobalt et/ou de nickel métallique,
b) on passive les particules de cobalt et/ou de nickel métallique par traitement par un gaz inerte contenant de l'oxygène moléculaire,
c) on presse les particules de cobalt et/ou de nickel passivées avec le lubrifiant pour former des corps façonnés, et
d) on active les corps façonnés contenant les particules de cobalt et/ou de nickel passivées ainsi que le lubrifiant, par traitement par l'hydrogène à une température de 250 à 500°C.

8. Procédé selon la revendication 7, caractérisé en ce qu'on réduit les particules d'oxyde de nickel et/ou de cobalt jusqu'à un degré de réduction ⩾ 95 %.

9. Procédé selon les revendications 7 et 8, caractérisé en ce qu'on ne descend pas en dessous d'un degré de réduction de 80 % lors de la passivation des particules de cobalt et/ou de nickel métallique.

10. Procédé selon les revendications 7 à 9, caractérisé en ce qu'en outre, on passive les corps façonnés contenant les particules de cobalt et/ou de nickel ainsi que le lubrifiant au moins une fois par traitement par un gaz inerte contenant de l'oxygène moléculaire et ensuite on les active par traitement par l'hydrogène à une température de 250° à 500° C.

11. Procédé selon les revendications 1 à 10, caractérisé en ce qu'on utilise des alcanenitriles ou des alcanedinitriles ayant de 3 à 18 atomes de carbone comme matières premières.